# EUROPEAN PATENT SPECIFICATION

(11) **EP 3 995 735 B1**
(45) Date of publication and mention of the grant of the patent: **05.04.2023**
(21) Application number: 21206655.9
(22) Date of filing: 05.11.2021
(51) Int. Cl.: F21S 6/00, F21W 121/00

(54) **ELECTRONIC CANDLE**
ELEKTRONISCHE KERZE
BOUGIE ÉLECTRONIQUE

(30) Priority: 09.11.2020 CN 202011235326
(43) Date of publication of application: 11.05.2022
(73) Proprietor: Cao, Liling, 518116 Shenzhen, Guangdong (CN)
(72) Inventor: Cao, Liling, 518116 Shenzhen, Guangdong (CN)
(74) Representative: Canzler & Bergmeier Patentanwälte Partnerschaft mbB

(56) References cited:
- EP-A1- 3 848 624
- WO-A1-2014/165932
- CN-A- 108 071 967
- US-A1- 2017 108 188
- US-A1- 2017 326 574
- US-A1- 2018 003 352

## Description

### TECHNICAL FIELD

The present disclosure relates to electronic candles.

### BACKGROUND ART

Electronic candles are widely favored by consumers because of their environmental protection and energy conservation. Now there are electronic candles with an aromatherapy function, either through an atomizer that atomizes an aromatic liquid and sprays it out, or by adding aromatic liquid or solid to make the aroma volatilize. The first type of aromatherapy candle has poor candle simulation effect, complex structure, and large consumption of aromatherapy raw materials is required. The second type of aromatherapy candle needs to be heated, generally by heat generated by the circuit board itself, and the aromatherapy effect is not good.

The patent application US 2017/0326574 A1 discloses an electronic fountain candle. The electronic fountain candle includes a candle body, where a chamber is formed in the candle body, a stepped water storage tank is arranged in the chamber, and a cover plate is connected to steps of the water storage tank; a first through hole and a plurality of second through holes are formed in the cover plate, a top of the first through hole is higher than an upper surface of the cover plate, a plurality of first light emitting diodes are arranged on two sides of the top of the first through hole, and a bottom of the first through hole is lower than a lower surface of the cover plate and is connected with a water pump; tops of the second through holes are higher than the upper surface of the cover plate; and a power unit.

### SUMMARY

The present disclosure is intended to provide an electronic candle which has an ingenious structure, a good simulation effect and saves materials.

It is provided an electronic candle. The electronic candle includes a housing, an electric wire extending out of an upper end face of the housing, a flame piece connected to a tail end of the electric wire and a control circuit arranged in the housing and electrically connected with the electric wire. At least a middle portion of the upper end face of the housing is formed as a concaved portion, holes are provided in the concaved portion, a container configured for containing liquid is arranged below the holes. An opening of the housing is connected with the upper end face of the housing in a sealed manner, the holes are communicated with an interior of the container

According to the invention, the electronic candle further includes a driving mechanism electrically connected with the control circuit; and an actuating mechanism driven by the driving mechanism; the actuating mechanism is configured to squeeze and loosen the container under driving of the driving mechanism.

In some embodiments, the driving mechanism includes a motor, the actuating mechanism includes a lead screw connected to an output shaft of the motor and a nut seat connected to a nut of the lead screw, and when the electronic candle is normally vertically placed, the nut seat is positioned right below the container. When the motor rotates, the nut of the lead screw is driven to move up and down on a body of the lead screw, and then the nut seat is driven to squeeze and loosen the container.

In some embodiments, the control circuit includes a remote signal receiving device

In some embodiments, the nut of the lead screw is a cylindrical nut, the nut seat is fixed on an end of the cylindrical nut and has a supporting surface which abuts against the bottom of the container.

In some embodiments, the electronic candle further includes a position-limit mechanism for limiting a maximum actuation position and a minimum actuation position of the actuating mechanism.

In some embodiments, the position-limit mechanism includes: a first conductive sheet synchronously moving with the nut seat; a second conductive sheet arranged at the maximum actuation position; and a third conductive sheet arranged at the minimum actuation position. The first conductive sheet, the second conductive sheet and the third conductive sheet are electrically connected with the control circuit

In some embodiments, a fixing ring is sleeved on a bottom of the container or a fixing claw is clamped on the bottom of the container, a protruding portion extending outwards is formed on the fixing ring or the fixing claw, and the first conductive sheet is fixed on the protruding portion.

In some embodiments, a fixing ring or a fixing claw surrounding the bottom of the container is provided on the nut seat, a protruding portion extending outwards is formed on the fixing ring or the fixing claw, and the first conductive sheet is fixed on the protruding portion.

In some embodiments, the electronic candle further includes an inner cover or a support frame arranged outside the driving mechanism, the actuating mechanism and the container. A gap for the protruding portion to extend through is formed in the inner cover or the support frame, and the second conductive sheet and the third conductive sheet are fixed on the inner cover or the support frame.

In some non-claimed alternative embodiments, the electronic candle includes a knob fixed at a bottom of the housing, a lead screw arranged in the housing and connected with the knob and a nut seat connected to the nut of the lead screw When the electronic candle is normally vertically placed, the nut seat is positioned right below the container and abuts against the bottom of the container. When the knob is rotated in a positive direction or a reverse direction, the nut of the lead screw is driven to move upwards or downwards on a body of the lead screw, and then the nut seat is driven to squeeze and loosen the container.

A concaved portion is provided on the upper end face of the housing of the electronic candle. Holes are provided in the concaved portion, and communicated with an internal space of the container, so that liquid can be accommodated in the concaved portion so as to simulate melted wax water generated on an upper end of a candle body after a real candle is burned, thereby having a high simulation effect. The liquid may be liquid containing fragrance, so as to realize a simulation candle with aromatherapy function. In real use, the contained may be filled with high concentration aromatic liquid, the liquid overflows to the concaved portion. After being used for a period of time, when the aromatic liquid in the concaved portion volatilizes, it can be used by adding water directly. When the concentration of the aromatic liquid decreases and the aromatherapy effect is not good, high concentration aromatic liquid can be added, thereby saving the aromatic liquid.

### BRIEF DESCRIPTION OF THE DRAWINGS

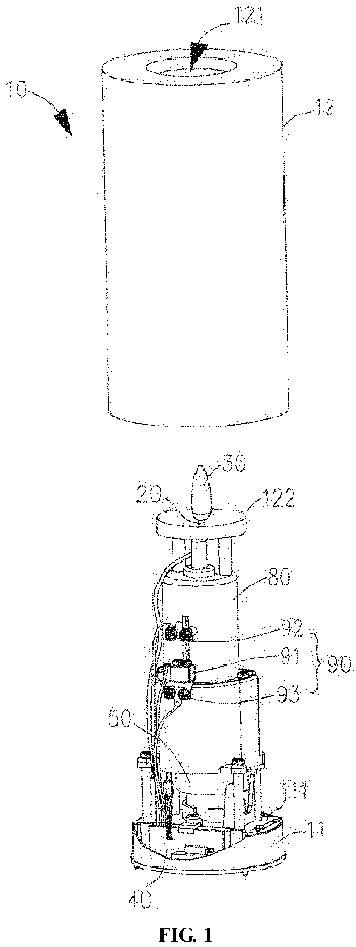

FIG. 1 is an exploded view of an electronic candle according to an embodiment of the present disclosure.

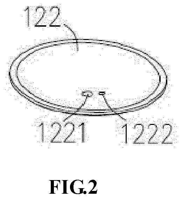

FIG. 2 is a schematic structural view of a tray of the electronic candle in FIG. 1.

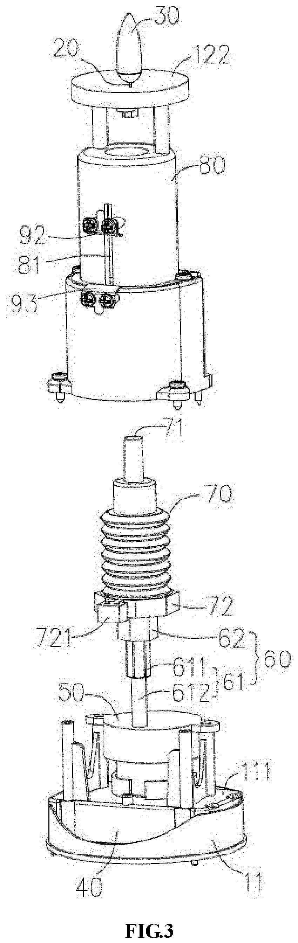

FIG. 3 is an exploded view of a part of an electronic candle in accordance with an embodiment of the present disclosure.
FIG. 4 is a schematic cross-sectional view of a flame piece of an electronic candle in accordance with an embodiment of the present disclosure.

### DETAILED DESCRIPTION OF THE EMBODIMENTS

The disclosure will now be described in detail through several embodiments with reference to the accompanying drawings.

As shown in FIG.s 1 to 3, in an embodiment of the present disclosure, an electronic candle mainly includes a housing 10, an electric wire 20 extending from an upper end of the housing 10, a flame piece 30 connected to a tail end of the electric wire 20, a control circuit 40 arranged in the housing 10 and electrically connected to the other end of the electric wire 20, a driving mechanism 50 electrically connected to the control circuit 40, and an actuating mechanism 60 driven by the driving mechanism 50, a container 70 for containing liquid, and an inner cover 80.

The housing 10 is substantially cylindrical and includes a base 11 and an upper cover 12 buckled on the base. The upper cover 12 is used for simulating a candle body of a candle, and may be made of paraffin wax in order to improve the simulation effect. The base 11 may be made of an electrically insulating material, such as plastic. An opening 121 is defined in a middle of an upper end face of the upper cover 12 and is sealed by an electrically insulating tray 122.

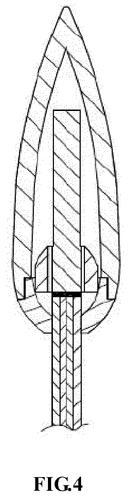

The tray 122 is preferably made of an environmentally friendly, non-toxic plastic or other polymeric material that is colored to conform to the upper cover 12. The tray 122 has a concaved shape for simulating a concaved portion formed by melting a top portion of the candle body after a real candle is burned. Two holes 1221, 1222 are formed in the tray 122, where the hole 1221 is located at a center of the tray and used as a wire hole, the electric wire 20 passes through the hole 1221, and a gap between the electric wire and the hole 1221 is sealed and waterproof. The electric wire 20 and its portion extending out of the housing 10 should be encased in a black hard outer skin to simulate color and state of a candle wick. A flame piece 30 is fixed to a tail end of the electric wire 20 for imitating a flame of a burning candle. The structure of the flame piece 30 is shown in FIG. 4, and has been disclosed by the Chinese utility model patent ZL 201620728691.1 (issued on January 18, 2017), the U.S. patent US 10436396 (issued on October 8, 2019) and the German utility model patent DE 202017103857.2 (issued on September 14, 2017), which is not further described here.

The hole 1222 is located adjacent the hole 1221. The container 70 is fixed below the hole 1222, and an opening 71 of the container 70 is hermetically connected to an upper inner surface of the tray 122 of the housing 10, such that the hole 1222 communicates only with the inner space of the container 70. The liquid inside the container 70 can only flow out and in through the hole 1222. In the present embodiment, the container 70 is a stretchable container which can be stretched and shrank in a height direction of the electronic candle, the upper end of the container 70 extends toward the upper end face of the housing so as to form a tube, and an opening 71 is defined in a top end of the tube. The top end of the tube is hermetically connected to the periphery of the hole 1222 of the tray 122. The container 70 is preferably made of an environmentally friendly, non-toxic material, such as a polypropylene material, for containing an aromatic liquid.

The driving mechanism 50 is used to drive the actuating mechanism 60 to perform a squeezing action and a loosening action on the container 70 to squeeze liquid from the container 70 onto the tray 122 or to draw liquid from the tray 122 back into the container 70.

In this embodiment, the driving mechanism 50 is a micro direct current motor fixed on the base 11 of the housing 10. In order to power the motor, the base 11 is provided with a battery holder 111 with an opening facing outwards, i.e. towards the underside of the electronic candle. The control circuit 40 is fixed to the outer side wall of the battery holder 111, and the driving mechanism 50 and the inner cover 80 are fixed to a side of the bottom of the battery holder facing the upper cover 12. The drive shaft of the driving mechanism 50 extends toward and out of the upper end of the upper cover.

The actuating mechanism 60 includes a lead screw 61 connected to the drive shaft of the driving mechanism 50 and a nut seat 62 connected to a nut 611 of the lead screw 61. A body 612 of the lead screw 61 is parallel to the axis of the electronic candle, the nut 611 is a cylindrical nut, and the nut seat 62 is fixed to one end of the cylindrical nut. When the motor of the driving mechanism 50 rotates in a first direction or a second direction opposite to the first direction, the body 612 rotates therewith, driving the nut 611 to move up or down along the body 612, thereby further driving the nut seat 62 to move up or down. In the present embodiment, when the electronic candle is normally vertically placed, in the set initial state, the nut seat 62 is located at the lowest position, which is located directly below the container 70 and comes into contact with (abuts against) the bottom of the container 70. As the nut seat 62 moves upward, the container 70 is squeezed. When the motor reverses rotation to drive the nut seat 62 to move downward after the squeezing action is performed, the container is relaxed, and the inner space of the container becomes larger. The cylindrical nut 611 allows the end portion of the body 612 of the lead screw 61 not to protrude from the top end portion of the cylindrical nut 611 during operation of the motor. In this embodiment, the nut seat 62 is a hexagonal nut fixed to the upper end of the cylindrical nut 611, and the end face thereof serves as a support surface for the container 70.

A position-limit mechanism 90 is provided to prevent the actuating mechanism 60 from over-squeezing or loosening the container 70. That is, the position-limit mechanism 90 serves to limit the maximum (highest) and minimum (lowest) actuation positions of the actuating mechanism 60. In the embodiment, the position-limit mechanism 90 includes a first conductive sheet 91 synchronously moving with the nut seat 62, a second conductive sheet 92 arranged at the maximum actuation position and a third conductive sheet 93 arranged at the minimum actuation position. The first to third conductive sheets 91, 92, 93 are electrically connected to the control circuit 40. When the first conductive sheet 91 and the second conductive sheet 92 are in electrical contact, the motor stops driving the nut seat 62 upward, and when the first conductive sheet 91 and the third conductive sheet 93 are in electrical contact, the motor stops driving the nut seat 62 downward.

In order to fix the position-limit meaning 90, a fixing ring 72 is sleeved on the outer periphery of the bottom of the container 70, and a protruding portion 721 extending and protruding outwards is formed on the fixing ring 72. The first conductive sheet 91 is fixed to the protruding portion 721. And the first conductive sheet 91 is bent to be generally C-shaped so that the second conductive sheet 92 and the third conductive sheet 93 can be contacted from the upper and lower sides, respectively. The second conductive sheet 92 and the third conductive sheet 93 are both fixed to the outer surface of the inner cover 80. In this embodiment, the inner cover 80 covers the outside of the driving mechanism 50, the actuating mechanism 60 and the container 70, and in order for the protruding portion 721 of the fixing ring 72 to protrude out of the inner cover 80, the inner cover 80 is formed with a gap 81 as a vacancy-avoiding space on the path in which the protruding portion 721 moves. The tray 122 is also fixed to the inner cover 80 by posts to facilitate assembly and fixing.

When the switch of the electronic candle is turned on, the flame piece 30 emits light, the driving mechanism 50 synchronously starts to work, the actuating mechanism 60 is driven to squeeze the container 70, the liquid in the container 70 is slowly squeezed into the concaved portion (i.e. the tray 122) of the upper end of the housing 10, and when the liquid level in the concaved portion reaches the preset position (i.e. when the first conductive sheet of the actuating mechanism 60 contacts the second conductive sheet); the driving mechanism stops working. When the switch of the electronic candle is turned off, the flame piece 30 is extinguished, the drive motor drives the actuating mechanism to reset until the first conductive sheet contacts the third conductive sheet, the container 70 is slowly released and finally restored to the original state, and the liquid in the concaved portion of the upper end of the housing is slowly drawn back into the container. Therefore, the phenomenon that molten wax water is formed at the upper end of the candle body when the candle burns, and the candle water solidifies and seems to disappear after the candle is extinguished is vividly simulated. In addition, when the liquid is fragrant liquid, it can send out fragrance on the tray to achieve the effect of aromatherapy. When not in use, the liquid is drawn back, which can be used for a long time, with less consumable materials and low use cost. Replacing and replenishing the fragrant liquid is convenient and easy to operate. By controlling the working speed of the driving mechanism, adjusting the speed of liquid outlet and the speed of liquid withdrawal, the simulation effect is better.

In addition, the control circuit may further include a remote-control signal receiving device so that the electronic candle may be remotely controlled using a remote controller. When multiple simulation candles are simultaneously used, a single remote controller may control multiple candles at the same time, and the use is more convenient. The remote-control signal receiving device may be an infrared signal receiving device or a wireless radio frequency signal receiving device.

In the embodiments described above, only a single switch may be used to simultaneously control the flame piece and the driving mechanism. It will be understood that in other embodiments, multiple switches may be used to separately control the flame piece and the driving mechanism.

In addition, in alternate non claimed embodiments, the driving mechanism and actuating mechanism may be omitted. If the phenomenon of wax water generated when the candle is burnt is simulated, water can be directly poured at the concaved portion of the upper end face of the housing. The inner container can be used for storing concentrated aromatic liquid, and the purpose of aromatherapy is realized by adding water directly from the top to dilute and release the aromatic liquid when being used. After being used for a period of time, when the aromatic liquid in the concaved portion volatilizes, it can be used by adding water directly. When the concentration of the aromatic liquid decreases and the aromatherapy effect is not good, high concentration aromatic liquid can be added, thereby saving the aromatic liquid.

It is understood that in other alternative not claimed embodiments, the driving mechanism may be eliminated, and a knob may be provided in a lower portion of the base of the housing, the other end of which is connected to a screw end of the lead screw. When the knob is rotated in the positive direction or the reverse direction, the lead screw is driven to rotate, the nut seat is moved upwards or downwards, the container is squeezed and loosened, and the function that liquid in the container flows into the tray or is sucked back into the container is achieved.

In the embodiment, since the candle body is thick, only the tray 122 is concaved, and it is understood that in other embodiments, when the candle body is thin, the upper end face of the upper cover 12 may be integrally concaved after the tray 122 is assembled. Or the upper end face of the upper cover is formed by a tray.

In the embodiment, the container is a collapsible container that can be extended and retracted in the direction of the height of the electronic candle, and it is understood that in other embodiments, the container may be made of a soft, flexible material. As long as the container is deformable when the actuating mechanism moves upwards, the liquid in the container can be squeezed out. In other embodiments, the tube of the container may be omitted.

In other embodiments, the inner cover may be replaced by a support frame made of plates or/and posts so long as the actuating mechanism, the position-limit mechanism, and the tray are fixed in predetermined positions. It is understood that the inner cover or support frame may also be eliminated and the actuating mechanism and the position-limit mechanism may be fixed to an inner wall of the upper cover.

In other embodiments, a generally C-shaped fixing claw may be used in place of the fixing ring to clamp the bottom of the container.

In other embodiments, the fixing ring may be fixedly connected to the nut seat and is integrally cup-shaped as part of the nut seat to support the bottom of the container. That is, a fixing ring or a fixing claw surrounding the bottom of the container is arranged on the nut seat, a protruding portion extending outwards is formed on the fixing ring or the fixing claw, and the first conductive sheet is fixed on the protruding portion, and the same function can be realized.

It is understood that in other embodiments, the driving mechanism may include an air cylinder. The actuating mechanism may include a support block connected to the tail end of the cylinder rod in place of the nut seat. During operation, the air cylinder pushes the air cylinder rod to move upwards to drive the support block to squeeze the container upwards. As the cylinder rod retracts, the support block moves downward, releasing the container. In order to prevent liquid splashing, buffer unit, such as springs or shrapnel, may be provided between the cylinder rod and the support block so that the support block moves relatively gently during operation.

It is understood that in other embodiments, more complex mechanical mechanisms may be used to replace the actuating mechanisms of the previous embodiments for the purpose of squeezing and releasing the container.

It is understood that in other embodiments, other position sensing devices, such as photoelectric sensor, may be used in place of the position-limit mechanism to achieve a constant movement path for the actuating mechanism.

In describing the present disclosure, it is to be understood that terms indicating orientation or positional relationship, such as "upper", "lower", "front", "rear", "left", "right", "vertical", "horizontal", "top" , "bottom", "inner", "outer", is based on the orientation or positional relationship shown in the drawings for the purpose of describing the disclosure and simplifying the description only, and is not intended to indicate or imply that the referenced device or element must have a particular orientation, be constructed and operated in a particular orientation, and therefore should not be construed as limiting the disclosure.

In addition, the terms "first" and "second" are only used for descriptive purposes, and are not intended to indicate or imply relative importance or to implicitly indicate the number of indicated technical features. Thus, the features defined with "first" and "second" may explicitly or implicitly include one or more of these features. In the description of the present disclosure, "a plurality of" means two or more, unless otherwise specifically defined.

In the present disclosure, unless otherwise clearly specified and limited, the terms "installing", "connecting", "fixing" and other terms should be understood in a broad sense. For example, it may be a fixed connection or be a detachable connection or be integrated in one-piece; it may be a mechanical connection or an electrical connection; it may be directly connected or indirectly connected through an intermediate medium, and it may be an internal communication between two components or an interaction relationship between two components. For those of ordinary skill in the art, specific meanings of the above-mentioned terms in the present disclosure can be understood according to specific circumstances.

## Claims

1. An electronic candle, comprising:
a housing (10), an electric wire (20) extending out of an upper end face of the housing (10), a flame piece (30) connected to a tail end of the electric wire (20) and a control circuit (40) arranged in the housing (10) and electrically connected with the electric wire (20), wherein
at least a middle portion of the upper end face of the housing (10) is formed into a concaved portion (122), a hole (1222) is provided in the concaved portion (122), a container (70) configured for containing liquid is arranged below the hole (1222); an opening (71) of the container (70) is connected with the upper end face of the housing (10) in a sealed manner, the hole (1222) is communicated with an interior of the container (70);
wherein
the electronic candle further comprises:
a driving mechanism (50) electrically connected with the control circuit (40); and an actuating mechanism (60) driven by the driving mechanism (50);
**characterized in that**
the actuating mechanism (60) is configured to squeeze and loosen the container (70) under driving of the driving mechanism (50).

2. The electronic candle according to 1, **characterized in that**, the driving mechanism (50) comprises a motor, the actuating mechanism (60) comprises a lead screw (61) connected to an output shaft of the motor and a nut seat (62) connected to a nut (611) of the lead screw (61), and when the electronic candle is normally vertically placed, the nut seat (62) is positioned right below the container (70); when the motor rotates, the nut (611) of the lead screw (61) is driven to move up and down on a body of the lead screw (61), and then the nut seat (62) is driven to squeeze and loosen the container (70).

3. The electronic candle according to 1, **characterized in that**, the control circuit (40) comprises a remote signal receiving device.

4. The electronic candle according to 2, **characterized in that**, further comprising a position-limit mechanism (90) for limiting a maximum actuation position and a minimum actuation position of the actuating mechanism (60).

5. The electronic candle according to 4, **characterized in that**, the position-limit mechanism (90) comprises:
a first conductive sheet (91) synchronously moving with the nut seat (62);
a second conductive sheet (92) arranged at the maximum actuation position; and
a third conductive sheet (93) arranged at the minimum actuation position;
wherein the first conductive sheet (91), the second conductive sheet (92) and the third conductive sheet (93) are electrically connected with the control circuit (40).

6. The electronic candle according to 5, **characterized in that**, a fixing ring (72) is sleeved on a bottom of the container (70) or a fixing claw is clamped on the bottom of the container (70), a protruding portion (721) extending outwards is formed on the fixing ring (72) or the fixing claw, and the first conductive sheet (91) is fixed on the protruding portion (721).

7. The electronic candle according to 5, **characterized in that**, a fixing ring (72) or a fixing claw surrounding the bottom of the container (70) is provided on the nut seat (62), a protruding portion (721) extending outwards is formed on the fixing ring (72) or the fixing claw, and the first conductive sheet (91) is fixed on the protruding portion (721).

8. The electronic candle according to claim 6 or 7, **characterized in that** ,further comprising an inner cover (80) or a support frame arranged outside the driving mechanism (50), the actuating mechanism (60) and the container (70), a gap (81) for the protruding portion (721) to extend through is formed in the inner cover (80) or the support frame, and the second conductive sheet (92) and the third conductive sheet (93) are fixed on the inner cover (80) or the support frame.

## Patentansprüche

1. Elektronische Kerze, umfassend:
ein Gehäuse (10), einen elektrischen Draht (20), der sich aus einer oberen Endfläche des Gehäuses (10) erstreckt, ein Flammenteil (30), verbunden mit einem Endstück des elektrischen Drahtes (20), und eine Steuerschaltung (40), angeordnet in dem Gehäuse (10) und elektrisch mit dem elektrischen Draht (20) verbunden, wobei
mindestens ein mittlerer Teil der oberen Endfläche des Gehäuses (10) zu einem konkaven Bereich (122) ausgebildet ist, ein Loch (1222) in dem konkaven Bereich (122) vorgesehen ist, ein Behälter (70) zum Aufnehmen von Flüssigkeit unterhalb des Loches (1222) angeordnet ist; eine Öffnung (71) des Behälters (70) in einer abgedichteten Weise mit der oberen Endfläche des Gehäuses (10) verbunden ist, das Loch (1222) mit einem Inneren des Behälters (70) in Verbindung steht;
wobei
die elektronische Kerze ferner umfasst:
eine Antriebsvorrichtung (50), mit der Steuerschaltung (40) elektrisch verbunden, und eine Betätigungsvorrichtung (60), angetrieben durch die Antriebsvorrichtung (50);
**dadurch gekennzeichnet, dass** die Betätigungsvorrichtung (60) ausgelegt ist, den Behälter (70) unter dem Antrieb durch die Antriebsvorrichtung (50) zu quetschen und freizugeben.

2. Elektronische Kerze nach Anspruch 1, **dadurch gekennzeichnet, dass** die Antriebsvorrichtung (50) einen Motor umfasst, die Betätigungsvorrichtung (60) eine Gewindespindel (61), verbunden mit einer Abtriebswelle des Motors, und einen Mutternsitz (62), verbunden mit einer Mutter (611) der Gewindespindel (61), umfasst, und wenn die elektronische Kerze normal vertikal aufgestellt wird, der Mutternsitz (62) direkt unterhalb des Behälters (70) angeordnet ist; wenn sich der Motor dreht, wird die Mutter (611) der Gewindespindel (61) angetrieben, sich auf einem Hauptteil der Gewindespindel (61) auf und ab zu bewegen, und dann wird der Mutternsitz (62) angetrieben, den Behälter (70) zu quetschen und freizugeben.

3. Elektronische Kerze nach Anspruch 1, **dadurch gekennzeichnet, dass** die Steuerschaltung (40) eine Fernsteuersignal-Empfangsvorrichtung umfasst.

4. Elektronische Kerze nach Anspruch 2, **dadurch gekennzeichnet, dass** sie ferner eine Positionsbegrenzungsvorrichtung (90) zum Begrenzen einer maximalen Betätigungsposition und einer minimalen Betätigungsposition der Betätigungsvorrichtung (60) umfasst.

5. Elektronische Kerze nach Anspruch 4, **dadurch gekennzeichnet, dass** die Positionsbegrenzungsvorrichtung (90) umfasst:
eine erste leitfähige Platte (91), die sich synchron mit dem Mutternsitz (62) bewegt;
eine zweite leitfähige Platte (92), angeordnet an der maximalen Betätigungsposition; und
eine dritte leitfähige Platte (93), angeordnet an der minimalen Betätigungsposition;
wobei die erste leitfähige Platte (91), die zweite leitfähige Platte (92) und die dritte leitfähige Platte (93) mit der Steuerschaltung (40) elektrisch verbunden sind.

6. Elektronische Kerze nach Anspruch 5, **dadurch gekennzeichnet, dass** ein Befestigungsring (72) auf einen unteren Teil des Behälters (70) gehülst ist oder eine Befestigungsklaue auf den unteren Teil des Behälters (70) geklemmt ist, ein nach außen ragender vorspringender Teil (721) auf dem Befestigungsring (72) oder der Befestigungsklaue ausgebildet ist und die erste leitfähige Platte (91) auf dem vorspringenden Teil (721) befestigt ist.

7. Elektronische Kerze nach Anspruch 5, **dadurch gekennzeichnet, dass** ein Befestigungsring (72) oder eine Befestigungsklaue, der/die den unteren Teil des Behälters (70) umgibt, auf dem Mutternsitz (62) vorgesehen ist, ein nach außen ragender vorspringender Teil (721) auf dem Befestigungsring (72) oder der Befestigungsklaue ausgebildet ist und die erste leitfähige Platte (91) auf dem vorspringenden Teil (721) befestigt ist.

8. Elektronische Kerze nach Anspruch 6 oder 7, **dadurch gekennzeichnet, dass** sie ferner eine innere Abdeckung (80) oder eine Trägerrahmen umfasst, angeordnet außerhalb der Antriebsvorrichtung (50), der Betätigungsvorrichtung (60) und des Behälters (70), ein Spalt (81), damit sich der vorspringende Teil (721) dort hindurch erstreckt, in der inneren Abdeckung (80) oder dem Trägerrahmen ausgebildet ist und die zweite leitfähige Platte (92) und die dritte leitfähige Platte (93) auf der inneren Abdeckung (80) oder dem Trägerrahmen befestigt sind.

## Revendications

1. Bougie électronique, comprenant :
un boîtier (10), un fil électrique (20) s'étendant hors d'une face d'extrémité supérieure du boîtier (10), une pièce de flamme (30) connectée à une extrémité de queue du fil électrique (20) et un circuit de commande (40) disposé dans le boîtier (10) et connecté électriquement au fil électrique (20), dans laquelle
au moins une partie médiane de la face d'extrémité supérieure du boîtier (10) est formée en une partie concave (122), un trou (1222) est prévu dans la partie concave (122), un récipient (70) configuré pour contenir un liquide est agencé sous le trou (1222) ; une ouverture (71) du récipient (70) est reliée à la face d'extrémité supérieure du boîtier (10) de manière étanche, le trou (1222) est en communication avec un intérieur du récipient (70) ;
dans laquelle la bougie électronique comprend en outre :
un mécanisme d'entraînement (50) connecté électriquement au circuit de commande (40) ; et un mécanisme d'actionnement (60) entraîné par le mécanisme d'entraînement (50) ;
**caractérisée en ce que** le mécanisme d'actionnement (60) est configuré pour serrer et desserrer le récipient (70) sous l'effet de l'entraînement du mécanisme d'entraînement (50).

2. Bougie électronique selon la revendication 1, **caractérisée en ce que** le mécanisme d'entraînement (50) comprend un moteur, le mécanisme d'actionnement (60) comprend une vis-mère (61) reliée à un arbre de sortie du moteur et un siège d'écrou (62) relié à un écrou (611) de la vis-mère (61), et lorsque la bougie électronique est normalement placée verticalement, le siège d'écrou (62) est positionné juste en dessous du récipient (70) ; lorsque le moteur tourne, l'écrou (611) de la vis-mère (61) est entraîné pour se déplacer vers le haut et vers le bas sur un corps de la vis-mère (61), et ensuite le siège d'écrou (62) est entraîné pour serrer et desserrer le récipient (70).

3. Bougie électronique selon la revendication 1, **caractérisée en ce que** le circuit de commande (40) comprend un dispositif de réception de signaux à distance.

4. Bougie électronique selon la revendication 2, **caractérisée en ce qu'**elle comprend en outre un mécanisme de limitation de position (90) pour limiter une position d'actionnement maximale et une position d'actionnement minimale du mécanisme d'actionnement (60).

5. Bougie électronique selon la revendication 4, **caractérisée en ce que** le mécanisme de limitation de position (90) comprend :
une première feuille conductrice (91) se déplaçant de manière synchrone avec le siège d'écrou (62) ;
une deuxième feuille conductrice (92) disposée à la position d'actionnement maximale ; et
une troisième feuille conductrice (93) disposée à la position d'actionnement minimale ;
dans laquelle la première feuille conductrice (91), la deuxième feuille conductrice (92) et la troisième feuille conductrice (93) sont connectées électriquement au circuit de commande (40).

6. Bougie électronique selon la revendication 5, **caractérisée en ce qu'**une bague de fixation (72) est manchonnée sur un fond du récipient (70) ou une griffe de fixation est serrée sur le fond du récipient (70), une partie protubérante (721) s'étendant vers l'extérieur est formée sur la bague de fixation (72) ou la griffe de fixation, et la première feuille conductrice (91) est fixée sur la partie protubérante (721).

7. Bougie électronique selon la revendication 5, **caractérisée en ce qu'**une bague de fixation (72) est manchonnée sur un fond du récipient (70) ou une griffe de fixation est serrée sur le fond du récipient (62), une partie protubérante (721) s'étendant vers l'extérieur est formée sur la bague de fixation (72) ou la griffe de fixation, et la première feuille conductrice (91) est fixée sur la partie protubérante (721).

8. Bougie électronique selon la revendication 6 ou 7, **caractérisée en ce qu'**elle comprend en outre un couvercle intérieur (80) ou un cadre de support disposé à l'extérieur du mécanisme d'entraînement (50), du mécanisme d'actionnement (60) et du récipient (70), un espace (81) pour que la partie protubérante (721) s'étende à travers est formé dans le couvercle intérieur (80) ou le cadre de support, et la deuxième feuille conductrice (92) et la troisième feuille conductrice (93) sont fixées sur le couvercle intérieur (80) ou le cadre de support.
